# EUROPEAN PATENT APPLICATION

(11) **EP 1 340 556 A2**
(43) Date of publication of application: **03.09.2003**
(21) Application number: 03386004.0
(22) Date of filing: 03.03.2003
(51) Int. Cl.: B08B 7/00, B23K 26/14

(54) **A method and device for cleaning surfaces using temporarily coincidental laser pulses of two different wavelengths**

(30) Priority: 01.03.2002 GR 2002100116
(71) Applicant: Foundation for Research and Technology-Hellas (FO.R.T.H.), Institute of Electronic Structure and Laser, 711 10 Heraklion (Crete) (GR); Zafiropulos, Vassilis, 71201 Heraklion, Crete (GR)
(72) Inventor: Zafiropulos, Vassilis, 71201 Heraklion, Crete (GR); Pouli, Paraskevi, 71110 Heraklion, Crete (GR); Eglezis, Apostolis, 71110 Heraklion, Crete (GR); Petrakis, Aristides, 71110 Heraklion, Crete (GR)

(57) **Abstract**

The present invention relates to a method and a device for removal of any kind of superficial encrustation from surfaces, by the synchronous use of the fundamental and one of the harmonic frequencies of a short-pulse laser system (in the order of 1- 40 ns). This is embodied by temporal and spatial overlapping of the two beams in various ratios of energy density values between them. The present invention relates to a problem of major concern, related to the cleaning of stone-sculpted surfaces of historic and/or artistic value without any alteration to the underlying authentic surfaces. The invention can be employed in other materials such as façades, metallic surfaces, glass, paper, wood etc. as well as in other applications of industrial or medical interest, such as the removal of radioactive residues from any type of surfaces or the removal of deposits from any kind of animal tissues. The success of this method is based on the combined action of two discrete mechanisms for the removal of surface deposits, embodied by the use of laser pulses of two different wavelengths simultaneously. The suggested device is based on the spatial and temporal overlapping of the fundamental frequency with one of its harmonics, as well as the possibility to adjust the energy of each one of the two radiations individually.

## Description

The present invention relates to a method and device for removing undesirable surface layers. This is embodied by the simultaneous use of the fundamental and one of the harmonic frequencies of a short-pulse laser (in the order of 1 to 40 ns) in temporal and spatial overlapping and in various energy density ratios between them.

The present invention solves a major problem that relates to cleaning of stone-sculpted surfaces (of historic or/and artistic value) without any alteration to the underlying original surface. The invention can be also applied in different fields related to the conservation of historic and artistic cultural heritage, such as building facades, metal surfaces, glass, paper, wood etc., as well as in other industrial or medical applications, such as the removal of radioactive residues from all types of surfaces or the removal of deposits from all types of tissues, such as teeth etc.

Sculpted surfaces of historic or/and artistic value can be, but not limited, statues, sculpted relief representations, as for example friezes, pediments etc. architectural elements, as for example pillars, capitals etc. These can be made of, but not limited, stone (such as marble, limestone etc), ceramic materials (such as terracotta), plaster etc. Undesirable encrustation include, but not limited, superficial crusts originating from the object's exposure to the atmospheric pollution, fire etc., superficial crusts originating from the object's burial into the soil, biological attack or any combination of the above, etc. In most cases, the removal of the undesirable layers is necessary, without the destruction, removal, discoloration or in general alteration of the substrate. The substrate may be, but not limited, the authentic surface of the sculpted object, layers of prior treatments with historical value, pigments, the original sculpted surface which has been weathered prior to the deposition of the undesirable layers and therefore it retains information of the authentic sculpted surface, etc. The selection of the cleaning limit is a very important parameter in the conservation procedure and it is usually defined by rules imposed by science, archaeology and aesthetics.

The method of selective (locally, quantitatively and in certain cases qualitatively) removal of material from a surface by means of laser radiation is well known from several years and has several applications. Many of these applications are patent protected, such as:
- Cleaning of surfaces from radioactive residues (EP 0653762, 0678212).
- Selective removal of surface layers from ceramics (EP 0653395).
- Manufacture of superconductors' elements (EP 0698931).
- Manufacture of specific optical gratings (EP 0652400, 0636490, 0636491)
- Removal of undesirable material from surfaces, such as concrete or stone (EP 0380387 or WO 9007988).
- Removal of oxides from boat surfaces (WO 8301400).
- Removal of paint from surfaces of composite materials, mainly for use in aeronautic (EP 0391113, 0677402, WO9526255).
- Micro-scale surface engraving (WO 9527587).
- Cleaning of surfaces of equipment (#5637245).
- Removal of one or more surface layers from objects based on (a) the different absorption coefficient of each layer to certain wavelengths (WO 9846391) and (b) the application of a specific device (WO 9638257).
- Removal of plaque from teeth (WO 4818230, WO 5795153 etc.).
- An application of the above mentioned mechanism is the manufacturing of specific optical gratings by means of the fundamental radiation of a Nd:YAG laser at 1064 nm (EP 0652400, 0636490, 0636491).

The removal of inorganic material by means of short laser pulses, in the order of 1 to 40 ns, is achieved through two discrete mechanisms. These mechanisms depend on the wavelength of the applied pulsed laser radiation and on the percentage that this is absorbed from the layer to be removed and the substrate. In general terms, two different cases are distinguished:
A. In the case where the radiation is equally absorbed by the undesirable layer and the substrate, material removal is performed in discrete steps. For each "material"-"type of laser" combination the quantity of the removed material per single laser pulse can be experimentally calculated as a function of the power or the energy density of the applied laser radiation. The energy density is defined as the ratio of the energy of the laser pulse to the surface area of the laser-material interaction. This surface area depends on the distance of the focusing lens from the surface of the material. Thus it is possible to calculate precisely the energy density and the number of the laser pulses required in order to remove a layer of material of a certain thickness. In this case, the exact thickness of the material and the fluctuation it may have across the surface is of major importance in order to achieve a homogeneous surface cleaning.
B. In the case where the material to be removed absorbs the laser radiation of a certain wavelength more than does the substrate (the material to be kept intact), the energy flux threshold for the removal of the superficial layer is less than the energy flux required for the removal of the substrate material. In such case, appropriate focusing of the laser beam so that the energy density of the laser is found within a certain range of values (defined by these two energy thresholds) can ensure the selective removal of the unwanted layer without the slightest damage to the substrate. Such a way to control the cleaning process is preferred due to the "self-limiting" action of the laser beam and can be applied in certain combinations of substrate- encrustation, mainly for cleaning stone surfaces.

So far the removal of undesirable encrustation from sculpted stone surfaces of historic and artistic value is mainly performed by means of the fundamental wavelength radiation of a Q-switched Nd:YAG laser, which emits at 1064nm (EP 0380387, Boquillon-WO 5151134). The use of this specific radiation has been reserved, as in several cases the final surface appears to be slightly discolored towards yellow hue *(yellowing).* These cases include among others, ancient marble sculptures with dark encrustation due to their exposure to the atmospheric pollution. In these cases it was not favorable to remove the whole encrustation as a certain part of the encrustation layer retains information of the authentic underlying surface.

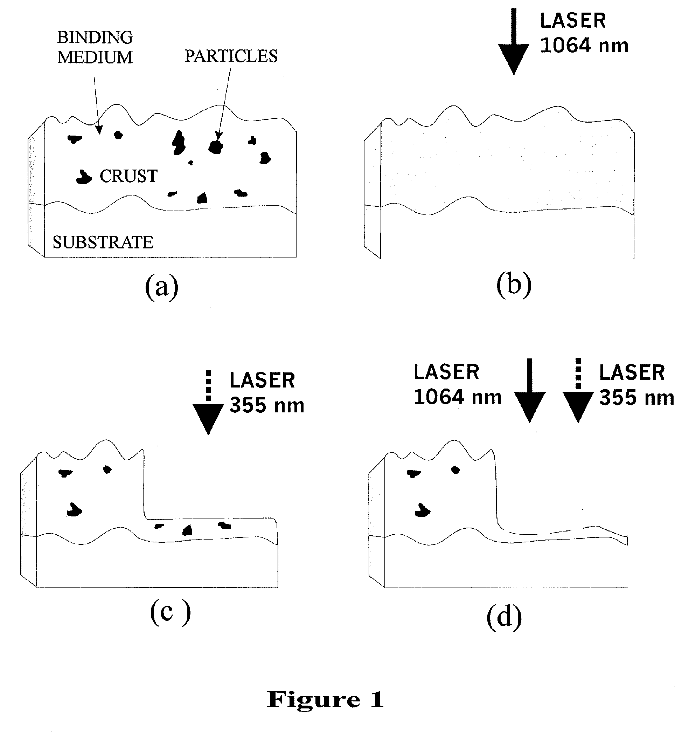

In order to understand the phenomenon it is necessary to briefly describe such undesirable superficial layers originating from the atmospheric pollution, as well as the mechanism responsible for their formation on the stone surface. These layers, widely known as *"black crusts",* can be found on the surface of stone or ceramic sculpted objects of historic and archaeological value, which are located outdoors and they are exposed to intense atmospheric pollution. Such superficial layers typically comprise, but not limited, airborne atmospheric particles, such as residues from the incomplete combustion of wood, coal, oil etc., pollen, dust, alumino-ferro-silicate salts and other particles which are transferred from the air masses and are deposited on the sculpted surface, especially on those areas sheltered from direct rain-washing. Bonding role for the consolidation of the above-mentioned airborne atmospheric particles on the stone surface has the re-crystallized or chemically transformed (e.g. gypsified) stone material. Its accumulation forms the suitable matrix for the development of such undesirable encrustation layers. A typical cross section of such a crust is schematically outlined in Figure 1a. The color and the hue of these dark-colored crusts may vary significantly due to the variety and the polymorphism in the composition and the concentration of the incorporated airborne atmospheric particles.

The removal of undesirable encrustation by means of the fundamental radiation of a Q-switched Nd:YAG laser (1064nm) is achieved through the mechanism B. It has been proved experimentally that the above-mentioned particles absorb this radiation on the average four times more intensively than the calcitic substrate (limestone, marble, plaster etc.) or the bonding medium (this may be, but not limited, gypsum, calcitic salts and alumino-ferro-silicate compounds). When the applied laser energy density, for certain pulse duration, is lower than the energy density threshold necessary to remove the crust's bonding material, *"selective vaporization"* of the embedded dark-colored airborne particles occurs, affecting slightly the main crust body. This particular case is schematically illustrated on Figure 1b. The scientific team of the inventors has proved that the void spaces resulting from the removal of the dark-colored particles as well as the change to the absorption spectra of the remaining encrustation may lead to the perception that the final surface is discolored *("yellowing").*

The removal of the undesirable layers by means of the third harmonic frequency of a Q-switched Nd:YAG laser (at 355nm) is achieved with the mechanism A. In this case, the various materials (particles and bonding material of the encrustation and/ or the substrate) absorb almost the same the ultraviolet laser radiation (at 355 nm) and thus it is possible to remove material from the total mass of the crust in a controllable way (spallation). Schematically this mechanism is illustrated in Figure 1c. The main advantage of this mechanism is that no discoloration phenomena are observed and no void spaces and morphological alterations are observed on the remaining surface. The disadvantage of this mechanism is the inability to control the removal process that is of major importance for these cases, as the exact thickness and the variation of the material to be removed are not known and may change considerably along the surface. As a result over-cleaning phenomena can be easily observed locally. Another significant disadvantage of this mechanism is the difficulty to remove homogeneously thick crusts, especially those originating from re-crystallization processes.

The objective of the present invention is the removal of undesirable superficial encrustation in a controlled way without discoloration or structural damage to the original surface. This objective has been achieved by the combined action of the two mechanisms for material removal mentioned above. This was accomplished by the simultaneous use of two laser beams of different wavelengths, whose pulses are temporally and spatially overlapped. The energy densities of the two beams are set to a certain ratio. The results of the synchronized action of the above mentioned mechanisms are schematically illustrated in Figure 1d, where the wavelengths used are 1064 and 355 nm.

Similar results can be obtained with the temporal and spatial use of various other wavelength combinations, such as 1064 and 266 nm or any infrared and any ultraviolet wavelength, provided that the duration of the laser pulses is shorter than 40ns. The infrared laser pulse originates from the fundamental frequency of a solid-state laser system while the ultraviolet pulse originates from the harmonic frequency, generated by a part of this infrared pulse. Thus, it is possible to achieve complete temporal overlapping of the two laser beams of a different wavelength. In this particular case, the wavelength of the laser pulses employed to clean sculpted stone surfaces were 1064 and 355 nm. The temporal and spatial overlapping of the two laser beams can be achieved by either separating one part of the fundamental beam, before this enters the harmonic generator, by means of a beam splitter, or by using the residual laser beam of the fundamental frequency after the harmonic generation procedure. The optimum energy ratio of the two radiations varies for each case of combination of wavelengths, as well as for each superficial encrustation and substrate. Finally, the area to be cleaned may, but not necessary, be moistened prior to cleaning, in order to enhance the cleaning efficiency.

The present invention uses the synchronized emission of laser pulses of fundamental wavelength, λ, and one of its harmonics, λ/n, in temporal and spatial overlapping and in various energy density ratios between them. It is also possible to use any of the two wavelengths independently, in case it is required from the specific application. In this manner it is possible to exploit the action of the two material removal mechanisms either independently or in combination. In this specific case, where stonework is cleaned by means of the temporal and spatial overlapping of the fundamental beam of a Q-switched Nd:YAG laser (λ=1064nm) and its third harmonic (λ= 355nm), all the problems associated with discoloring, or inhomogeneous and incomplete cleaning (in case where biological crust was also present on the surface), are prevented. Up to now these problems were responsible for the delay of the wide application of the laser cleaning method on stonework of historical and artistic value.

The developing procedure of the present invention contributed significantly to the understanding of the mechanisms responsible for the removal of the undesirable encrustation originating from the atmospheric pollution, from the surface of sculpted stone surfaces exposed to weathering.

The proposed device comprises a functional solution for the complete control of the combination of the two laser beams of different wavelengths (the fundamental and one of its harmonics). The device allows the modification of both the total energy of the system as well as the ratio of the energies of the two combined laser beams. The device is described further in reference to the attached drawings. Finally the present invention will be further explained in connection with two specific examples.

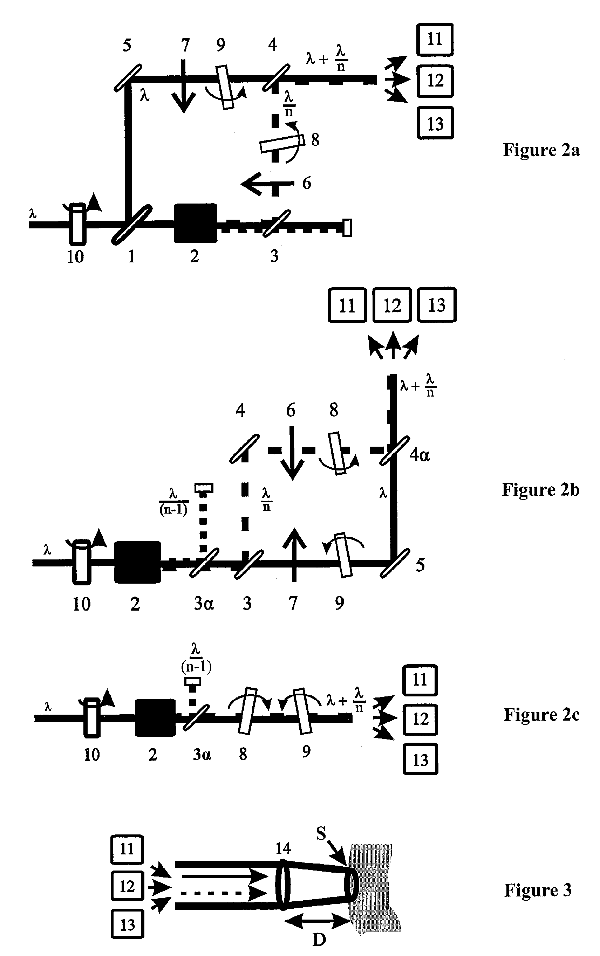

Figures 2a, 2b and 2c illustrate three variations of a device, embodiment of the present invention. Sequential events of the spatial and temporal overlapping of the fundamental and one of the harmonic wavelengths of a pulsed laser, in various ratios, are also illustrated, for clarity.

The schematically illustrated devices comprise a beam splitter 1 (of a certain reflectivity/ permeability ratio), an optical device for harmonic frequency generation 2, optical elements (3, 4, 4a and 5) for the alignment of the beams, controlled shutters 6, 7 for the selection of the exiting beams, attenuators 8, 9 and 10 for the adjustment of the laser beam energy and totally reflecting optical elements 3a for the dumping of the unused harmonic beams.

In particular, in the device outlined in Figure 2a the part of the laser beam, of the fundamental wavelength λ, which is about to spatially and temporally overlap at the exit with its harmonic radiation, of the wavelength *λ*/*n,* does not pass through the harmonic generation device 2. This way it is feasible to avoid possible changes to the beam's spatial profile.

A variation of the above-mentioned device is illustrated in figure 2b. In this case the essential condition is the top-hat profile of the fundamental beam when exiting the harmonic generators 2. The advantage of this version is the maximum possible energy efficiency in respect to the initial energy of the fundamental laser radiation.

Finally, figure 2c is a simpler variation of the one mentioned above; the selection of the independent or the combined emission of the two beams, as well as the possibility to control the ratio of their individual energies, can be done by rotation of the introduced optical attenuators 8 and 9. The advantage of this device is its simplicity due to the use of fewer elements.

The deriving double-wavelength laser beam can be delivered to the irradiated surface, but not limited, through an articulated optical arm 11, a fiber optic system 12 or any other beam- scanning device 13. The optical system 14, which may either partially focus or defocus or, in general, modify the cross-section of the two laser beams (Figure 3), allows the combined laser beams to interact with a surface of variable area S. As it is shown in Figure 3 the distance D between the optical system 14 and the irradiated surface can also be variable.

In the following preferred embodiments the present invention is outlined in more detail.

The invention exploits the possibility to temporally synchronize the laser pulses of fundamental and harmonic frequency. Temporal and spatial overlapping of the laser beam, of fundamental wavelength λ, with one of its harmonics, of wavelength *λ*/*n* (*n* integer, *n* ≥ 2), can be done, but not limited, through the devices schematically presented in Figures 2a, 2b and 2c.

The option to use either one of the two laser beams (of *λ*/*n* and λ wavelengths) independently or both of them in temporal and spatial overlapping, is possible, but not limited, with the externally controlled shutters 6 and 7 for *λ*/*n* and λ respectively (Figures 2a and 2b). In Figure 2c one of the two laser beams can be completely blocked either by rotation of the optical attenuators 8 and 9, or by introduction of a mirror, which reflects totally only one of the laser beams at 45° angle.

Separation and sequential re-alignment of the optical paths of the two beams, can be done, but not limited, by introduction of specially coated mirrors with high reflectivity for certain wavelengths at 45° angle. In this particular case the mirrors 3, 4, 4a are dielectrically coated to reflect the *λ*/*n* radiation, the mirror 3a the λ(n-1) radiation and the mirror 5 the λ radiation, all at 45° angle.

Modifications to the energy of each individual beam can be done, but not limited, with the introduction of two energy attenuators 8 and 9, for the *λ*/*n* and λ radiation respectively. Such scalar optical elements allow the continuous and controllable energy modification for each beam separately or together in combination. Intentional alteration to the energy ratio of the *λ*/*n* and λ laser beams, E(_{λ/n})/ E_{λ}, as well as to their absolute values, can be done by the appropriate selection of each attenuator's permeability values separately. Modification to the energy ratio E_{(λn)}/ E_{λ} is also possible by controlling the harmonic generation procedure. Rotation of the harmonic generation crystals (element 2), around a vertical or a horizontal axis, modulates the intensity of the generated laser beams with harmonic wavelengths, which is maximized or minimized for certain angles.

Modification to the total energy can be done, but not limited, by introducing internally into the laser system, an optical attenuator 10, which modifies the energy of the fundamental radiation, λ, prior to the harmonic generation. The total energy can also be altered by modification of the laser's oscillator and/or amplifier pumping procedure.

It is thus possible to select, at the output of the proposed device (figure 2a) or any of its variations (figures 2b and 2c), the emission of either the laser beam of fundamental wavelength λ, or one of it's fundamentals *λ*/*n,* as well as the spatial and temporal overlapping of the above-mentioned laser beams in various energy ratios.

The present invention will be further explained with reference to the following two examples that are specific applications (on specific energy density values) indented to illustrate, but in no way to restrict, the present application.

### EXAMPLE 1

This specific device has been used for the removal of the superficial "black encrustation" from the surface of ancient sculpted Pentelic marble. Figure 1a illustrates an example of a cross-section of a typical "black encrustation", consisting mainly of gypsum and secondarily from calcite and other Calcium (Ca) and Magnesium (Mg) salts. Airborne atmospheric particulates are also embedded in such crusts, the majority of them being dark-colored to black. Macroscopically such encrustations appear dark gray to black.

The device consists of a Q-switched Nd:YAG laser system emitting pulses of 6.5 ns at 1064nm. This system, which is able to emit at the second, third and fourth harmonic (at 532, 355 and 266 nm respectively), has been modified according to the Figure 2b, so that at its output, the fundamental beam wavelength (λ=1064 nm) and its third harmonic (*n*=3, λ/3= 355 nm) are temporally and spatially overlapped.

In this specific application it was chosen to adjust the ultraviolet radiation while keeping the infrared radiation constant. Therefore in the device illustrated in figure 2b the optical attenuator 9, which adjusts the energy of the fundamental radiation, has been placed vertically to the laser beam path (in order to get through the maximum fundamental radiation), while the optical attenuator 8, which adjusts the energy of the harmonic radiation, can be rotated in various angles in order to achieve the desirable energy ratios.

The exiting beam can be delivered, but not limited, through a mechanically articulated optical arm, which comprises of 6 mirrors, double-coated with dielectric coatings for high reflectivity at 1064 nm and 355 nm simultaneously. In this specific example and for certain total energy attenuation via the optical element 10, the maximum energy values, delivered at the output of the articulated arm, were 271 mJ and 127 mJ for the 1064 nm and 355 nm radiation respectively. The beam can be partially focused on the point of interaction by means of a converging lens placed at the output of the articulated arm (with focal point f=+30 cm).

The distance D between the converging lens and the sample (figure 3) determines the area S of the interaction surface, which is conversely proportional to the energy density, F = E S⁻¹. In this specific application the distance D ranged from 4 to 11.5 cm, and therefore the energy density of the fundamental radiation interacting with the surface, could be varied between 0.83 J/cm² and 1.43 J/cm², whereas the corresponding values for the energy density of the third harmonic were in the range of 0.39 J/cm² and 0.67 J/cm². The fact that the focal distances of the two beams with different wavelengths are slightly different is not considered critical as for this specific application it applies D « f (that is to say that the distance between the lens and the sample is always significantly shorter than the focal distance of the lens). This happens because the required laser energy densities onto the surface to be treated are usually of the same order of magnitude, or slightly higher than, the energy density of the unfocused laser beam. The difference between the surface areas of the cross-section of the two beams, away from the focal point is considered minimal (<5%).

By introducing the optical attenuator 8, which reduces the energy of the third harmonic, it is possible to modify the energy of the 355nm laser beam with respect to the incoming radiation's angle of gradient. In this particular application the energy of the third harmonic varied between 127 mJ (for angle = 0°) to 74 mJ (for angle = 24°), while the energy of the fundamental radiation was kept practically unchanged, 271 mJ (0° angle) - 262 mJ (24° angle). The combination of the attenuator 8 and the varying distance D, resulted in specific combinations of absolute energy density values for the two overlapping laser beams (1064 nm and 355 nm) capable for removing all the types of undesirable encrustation without any morphological alteration or discoloration to the underlying layer.

### EXAMPLE 2

This example is a variation of the application described in example 1. In this case the spatial and temporal overlapping of the fundamental and third harmonic radiation of a Q-switched Nd:YAG laser is realized through the device of Figure 2a. Given that the ratio of the energies necessary to remove the specific encrustation was approximately known, the beam splitter 1 was selected to have 40% reflectivity and 60% permeability. Thus the fundamental beam reaching the input of the articulated arm has a maximum energy of 340 mJ and a top-hat spatial profile as it originates from the fundamental laser beam. 60% of the fundamental beam passes through the harmonic generator 2 and generates ultraviolet radiation (355nm) with energy 74 mJ. The introduction of the infrared attenuator 9 adjusts the fundamental beam energy from 308 mJ (for 0° angle) to 11 mJ (for 25° angle). The alteration to the energy of the third harmonic beam was minor. The above-mentioned energies were measured at the output of the articulated optical arm. More specifically it ranges between 64mJ (at 0° angle) and 49mJ (at 25° angle). This way, as well as by alteration of the distance D, it was possible to achieve many combinations for the absolute values of the two laser beams (1064nm and 355nm) able to remove the various types of undesirable encrustation without any morphological alterations or discoloration to the underlying layer. It should also be noted that each different type of encrustation (which may vary significantly throughout area to be cleaned) requires a combination of particular energy fluencies of the two wavelengths.

In the following claims the symbols v, v', λ, λ' and *n* are determined as follows: The fundamental frequency of the laser, v, equals to the ratio c/λ, where c is the speed of the light and λ the wavelength of the fundamental laser radiation. The harmonic frequency of the laser, v', is equal to c/λ', where λ'= *λ*/*n* and *n* is an integer number, greater or equal to 2, n ≥ 2.

## Claims

1. A method for the removal of any type of superficial encrustation from surfaces using a pulsed laser with pulse duration shorter than 40ns. Such method is embodied by the temporal and spatial overlapping of the laser's fundamental beam, of frequency v, and one of its harmonics, of frequency v', in various ratios of energy density values between them. The method is comprising the steps of:
a) employing a pulsed laser system of wavelength λ and pulse duration shorter than 40ns
b) generating the *n*^{th} harmonic radiation *(n=* 2, 3, 4 etc.) of wavelength λ/*n*, with the use of non-linear optics.
c) spatial and temporal overlapping of the two laser beams with wavelength λ and λ/*n*.
d) adjusting the energy of each one of the beams, of wavelength λ and *λ*/*n,* separately, by using optical energy attenuators. Thus it is possible to achieve a certain energy ratio for optimum results.
e) delivering the total radiation towards the surface to be treated, by means of an optical system.
f) adjusting the energy density of the total radiation on the surface to be treated, by means of an optical system. Thus it is possible to achieve certain absolute values for the energy densities of the two radiations, of wavelengths λ and *λ*/*n.*
g) cleaning the surface by means of synchronous laser beams of λ and *λ*/*n* wavelength.
h) removing the laser beam away from the cleaned area

2. The method according to claim 1, wherein the materials to be cleaned belongs to the group which consists of sculpted stone surfaces of artistic value, sculpted stone surfaces of historical value, any kind of surfaces including the internal surface of tube-like surfaces with radioactive residue deposits, processed vegetal tissues (e.g. paper and wood), processed animal tissues (e.g. parchment), animal tissues, corneous tissues, glass, alloy, ceramic and composite materials.

3. The method according to claims 1 and 2, wherein the said laser is a Q-switched Nd:YAG laser with pulse duration shorter than 40ns, and where n=3. In the particular case, where the Q-switched Nd:YAG laser pulses with wavelength λ = 1064 nm and λ' = 355 nm and pulse duration of 6ns are synchronized to clean sculpted marble surfaces, the indicative values of the used energies are 200-500 mJ and 60-300 mJ, for λ and λ' respectively.

4. The method according to claims 1 and 2, wherein the said laser is a Q-switched Nd:YAG laser with pulse duration shorter than 40ns, and where n=4 (λ' = 266 nm).

5. The method according to claims 1 and 2, wherein the said laser is a Q-switched Nd:Glass laser with pulse duration shorter than 40ns.

6. The method according to claim 1, wherein the said laser is a Diode-pumped Nd:YAG laser with pulse duration shorter than 40ns.

7. The method according to claim 1, wherein the said laser is a tunnel laser.

8. Device for the removal of any kind of superficial encrustation from surfaces, embodied with the temporal and spatial overlapping of two laser beams with wavelength *λ*/*n* and λ, comprising of:
a) Pulsed laser of pulse duration shorter than 40ns and wavelength λ.
b) Beam splitter 1 of a certain reflectivity to permeability ratio of the laser beam of wavelength λ.
c) Optical system 2 for the generation of an harmonic frequency of wavelength *λ*/*n.*
d) Mirrors 3, 4 with dielectric coating for high reflectivity of the λ/n wavelength at 45°.
e) Mirror 5 with dielectric coating for high reflectivity of the λ wavelength at 45°.
f) Controlled shutters 6, 7 for the selection of the output laser beams λ/*n* and λ respectively.
g) Scalar optical systems 8, 9 for the adjustment of the energy of the output laser beams *λ*/*n* and λ respectively.
h) Optical system for the delivery of the *λ*/*n* and λ laser beams respectively.
i) Optical system 14 for the modification of the cross section of the laser beams *λ*/*n* and λ respectively, on a surface with variable surface area S.

9. The device according to claim 8, wherein a scalar optical system 10 has been introduced between the elements a) and b), in order to reduce the energy of the said laser beam with λ wavelength.

10. The device according to claims 8 and 9, wherein the said beam splitter 1 is absent. An optical system 3a, which dumps the laser beam with wavelength λ/(n-1) and a mirror 4a with dielectric coating for the high reflectivity of the λ/n radiation at 45° angle, have been introduced instead.

11. The device according to claim 10, wherein the said elements 3, 4, 5, 6 and 7 have been removed to simplify the device using less elements.

12. The device according to claims 8, 9 10 and 11, wherein the said optical system for the delivery of the radiation with wavelength λ/*n* and λ is an articulated optical arm 11 with mirrors specially coated to reflect highly the radiation λ and λ/*n* at 45° angles.

13. The device according to claim 12, wherein the said mirrors have an additional dielectric coating to highly reflect the radiation of wavelengths 400-700nm. This applies when an aiming beam is used to facilitate the system alignment.

14. The device according to claims 8, 9, 10 and 11, wherein the said optical system for the delivery of the radiation with wavelength *λ*/*n* and λ is a fiber optic system 12.

15. The device according to claims 8, 9, 10 and 11, wherein the said optical system for the delivery of the radiation with wavelength *λ*/*n* and λ is a mechanism, which scans the beam on the surface to be treated 13.
